# NEW EUROPEAN PATENT SPECIFICATION

(11) **EP 2 781 509 B2**
(45) Date of publication and mention of the opposition decision: **14.06.2023**
(45) Mention of the grant of the patent: 27.11.2019
(21) Application number: 14160639.2
(22) Date of filing: 19.03.2014
(51) Int. Cl.: A61K 31/55, C07D 223/16

(54) **New polymorph of ivabradine hydrochloride and method for its preparation**
Neues Polymorph von Ivabradinhydrochlorid und Verfahren zu dessen Herstellung
Nouveau polymorphe de chlorhydrate d'ivabradine et procédé pour sa préparation

(30) Priority: 19.03.2013 IT MI20130416; 24.04.2013 IT MI20130684
(43) Date of publication of application: 24.09.2014
(73) Proprietor: Chemo Research, S.L., 28050 Madrid (ES)
(72) Inventor: Barreca, Giuseppe, 23874 Montevecchia (IT); Gatti, Marco Maria, 28065 Cerano (IT); Ventimiglia, Gianpiero, 72021 Francavilla Fontana (IT)
(74) Representative: Palladino, Saverio Massimo

(56) References cited:
- WO-A1-2013/102919
- WO-A2-2011/098582
- US-A1- 2006 194 962
- US-A1- 2009 318 417
- US-A1- 2010 179 316
- US-B2- 7 176 197
- Stephen Byrn et al: "Pharmaceutical Solids: A strategic Approach to Regulatory Considerations", Pharmaceutical Research, vol. 12, no. 7, 1995, pages 945-954,
- International Journal of Pharmaceutics, 92 (1993), 157-166

## Description

### Field of the Invention

The present invention relates to a process for the preparation of a polymorph of ivabradine hydrochloride.

### State of the Art

Ivabradine (I), whose chemical name is 3-[3-({[(7S)-3,4-dimethoxybicyclo[4.2.0]octa-1,3,5-trien-7-yl]methyl}(methyl)amino)propyl]-7,8-dimethoxy-2,3,4,5-tetrahydro-1*H*-3-benzazepin-2-one, is a medicament used for the symptomatic treatment of angina pectoris.

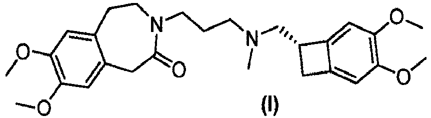

Ivabradine acts by reducing the heart rate with a mechanism different from that of beta blockers and from that of the calcium channel blockers, two classes of drugs normally used in the treatment of angina.

The preparation and therapeutic use of ivabradine and its salts with a pharmaceutically acceptable acid, in particular hydrochloric acid, have been described for the first time in European patent EP 0534859, according to which ivabradine hydrochloride is prepared by treating the free base with hydrochloric acid and then crystallizing from acetonitrile.

As well known to the skilled technician, the crystalline form and the morphology of the solid form of a pharmaceutical compound can significantly influence its physicochemical properties, for example its stability, the dissolution degree and bioavailability.

Some substances are known in a single crystalline form; others, instead, are known in two, three or even more crystalline forms. The property of some molecules or molecular complexes to assume more than one crystalline form or to remain amorphous, is normally defined polymorphism, and the different forms of a compound are defined polymorphs. In general, polymorphism is due to the ability of the molecules of a compound to change their forms, or to cause different inter-or intramolecular interactions, for example hydrogen bonds, which lead to different atomic arrangements in the crystal lattice of different polymorphs.

The different polymorphs of a substance possess different energies of the crystal lattice so that they show different physical properties such as shape, density, melting point, color, stability, dissolution degree or ability to be ground, granulated or compressed. These differences in morphology or polymorphism may also have a significant effect on the flowability of the ground solid (the flowability affects the ease with which a material is handled during its processing into a pharmaceutical product); on the stability during transport and storage of the unitary forms of administration; on the ability to produce different administration forms and their application; on the solubility in polar or non-polar, protic or aprotic solvents, in aqueous solutions, in gastric juices or serum; and finally on the bioavailability. From a physical point of view, once a salt of a pharmaceutical compound has been isolated, it can be characterized, for example, by analyzing its physico-chemical properties and/or its thermal behavior. The thermal behavior is normally determined in a laboratory, using the differential scanning calorimetry (DSC). Melting point, glass transitions, crystallinity, presence of solvates and/or polymorphs can be detected through their endo- or exothermic peaks, or by the variations of the base line in the corresponding DSC plot.

The different physico-chemical properties arising from polymorphism can instead be detected, for example, by X-ray diffraction of the powder (XRPD). Only crystalline samples diffract at well-defined angles, and peaks at different angles are observed depending on the nature of the crystalline form; therefore, each crystalline form gives rise to a unique powder diffraction pattern.

A first polymorphic form of ivabradine hydrochloride, defined α (alpha), has been described for the first time in U.S. Patent No. 7,176,197; according to the teachings of this patent, preparation of the polymorph requires a crystallization from a mixture of toluene and 1-methyl-2-pyrrolidinone.

The polymorphic form δ (delta) and a method for its preparation, on the other hand, are disclosed in U.S. Patent No. 7,384,932; while polymorph δd (delta-d) is disclosed in U.S. Patent No. 7,867,997.

According to what is described in these documents, crystallization of ivabradine hydrochloride is carried out in acetonitrile, producing form δ or form δd, depending on the drying conditions adopted.

Patent application US 2009/0318417 A1 discloses the γ (gamma) form of ivabradine hydrochloride.

Patent application US 2010/179316 A1 discloses a process for the preparation of ivabradine hydrochloride by treating ivabradine with alcoholic hydrogen chloride, and the amorphous form of ivabradine hydrochloride.

Other known polymorphic forms of ivabradine hydrochloride include form I, described in International patent application WO 2008/065681 and prepared by treating a solution of ivabradine in acetonitrile with a saturated solution of HCl in isopropanol; form ζ (Greek zed) described in International patent application WO 2012/025940 and prepared by crystallization of ivabradine hydrochloride from acetonitrile; and forms Z and X described in International patent application WO 2011/098582, which can be prepared, the former, by dissolving ivabradine in isopropanol and adding a saturated solution of HCl in isopropanol, and the latter by drying form Z.

Acetonitrile, as well as 1-methyl-2-pyrrolidinone or toluene, are solvents of class 2, according to the ICH classification. In accordance with the provisions in ICH Topic Q3C (R4) Impurities: Guideline for Residual Solvents, issued by EMEA in February 2009 (http://www.ema.europa.eu/docs/en_GB/document_library/Scientific_guideline/ 2009/09/WC500002674.pdf) the amount of said solvents in pharmaceutical products must be kept below precise values and should preferably be as low as possible. Accordingly, inclusion within a pharmaceutical form of a polymorph obtained directly by crystallization from a solvent (or a mixture of solvents) of class 2, according to the ICH classification, should preferably be avoided.

An improved process for the preparation of polymorphic forms δ and δd of ivabradine hydrochloride (in which the amount of residual acetonitrile is reduced to acceptable levels for inclusion in a pharmaceutical form) is described in International patent application WO 2013/017582. This process involves the preparation of an acetone solvate which is subsequently converted into the δd form by drying under *vacuum* at a temperature of 70 °C for 14 hours, or into the δ form by treatment in a humidity-controlled chamber for 72 hours. These processes, while being an improvement over the general state of the art, are not optimal for an industrial application, because, for example, they require a strict control of the relative humidity within the drying chamber, calling for the use of special equipment.

The provision of simple advantageous processes to obtain polymorphs of ivabradine hydrochloride, can be considered of remarkable interest in the field of pharmaceutics.

### Summary of the invention

These objects are achieved with the present invention, which relates to a process for the preparation of a polymorph of ivabradine hydrochloride according to the claims.

A first embodiment consists in a method for the preparation of the form ε of ivabradine hydrochloride according to claim 1
wherein in step a) the anhydrous ivabradine hydrochloride is employed in the form of one of the polymorphs α (alpha), δ (delta), δd (delta-d), I, ζ (Greek zed), X, or a mixture thereof.

Depending on the operating conditions (as described below), the above method for this embodiment of the present invention may optionally comprise, between steps b) and c), a step b') in which the mass obtained in step b) is cooled or allowed to cool down to a temperature between 20 and 25 °C.

A second embodiment consists in a method for the preparation of the form ε of ivabradine hydrochloride according to claim 6.

In a possible variant of this second embodiment, between steps d) and e) a further step d') is carried out, including seeding the solution obtained in step e) with the form ε of ivabradine hydrochloride; moreover, also in this case, depending on the operating conditions (as described below), this second embodiment can optionally comprise, between steps f) and g), a step f') in which the mass obtained in step f) is cooled or allowed to cool down to a temperature between 20 and 25 °C.

### Brief Description of the Figures

Figure 1 shows a XRPD spectrum of the form ε of ivabradine hydrochloride.
Figure 2 shows a FTIR spectrum of the form ε of ivabradine hydrochloride.
Figures 3 to 9 show the results of DSC tests carried out in different conditions on samples obtained according to the invention.

### Detailed Description of the Invention

The present inventors have unexpectedly found a method for the preparation of a polymorphic form of ivabradine hydrochloride.

All terms used in this application, unless otherwise specified, are to be understood in their ordinary meaning as known in the technical field. Other more specific definitions for certain terms used in this application are listed below and are intended to be applied uniformly to the entire application, unless otherwise indicated.

The term "about" includes the range of experimental error, which can normally occur when performing a measurement.

The term "mass" defines the combination of substrates, reagents, solvents, and products on which a physical or chemical transformation is carried out.

The term "seed" refers to a crystalline substance that is added to a solution of the same substance to induce its crystallization. Seeding with a specific crystalline form has often the useful effect of promoting crystallization of the substance in the same crystalline form of the seed.

The term "excipient" means any substance contained in the final pharmaceutical form other than the active ingredient and which generally may not be therapeutically effective by itself. Excipients are essential for the administration of the active substance, as they allow to deliver the drug to the target site. Excipients are commonly referred to as raw materials entering into the composition of a pharmaceutical preparation with the aim of giving a shape, to facilitate administration and preserve the active ingredient. Furthermore, they contribute to characterize the pharmaceutical preparation from the point of view of appearance, stability, biopharmaceutical profile and acceptability by the patient.

X-ray powder diffractometry (XRPD) and differential scanning calorimetry (DSC) were used to characterize the polymorphs of ivabradine hydrochloride obtained by the processes described in this application. The DSC measurements, referred to in the description and in the claims, were carried out in a nitrogen atmosphere; in addition, the value of the temperature ranges in which the thermal phenomena take place should be considered with a ± 0.5 °C accuracy.

Moreover, this polymorphic form is characterized by a FTIR spectrum comprising peaks at 3473, 2950, 1648, 1458 and 1305 cm⁻¹.

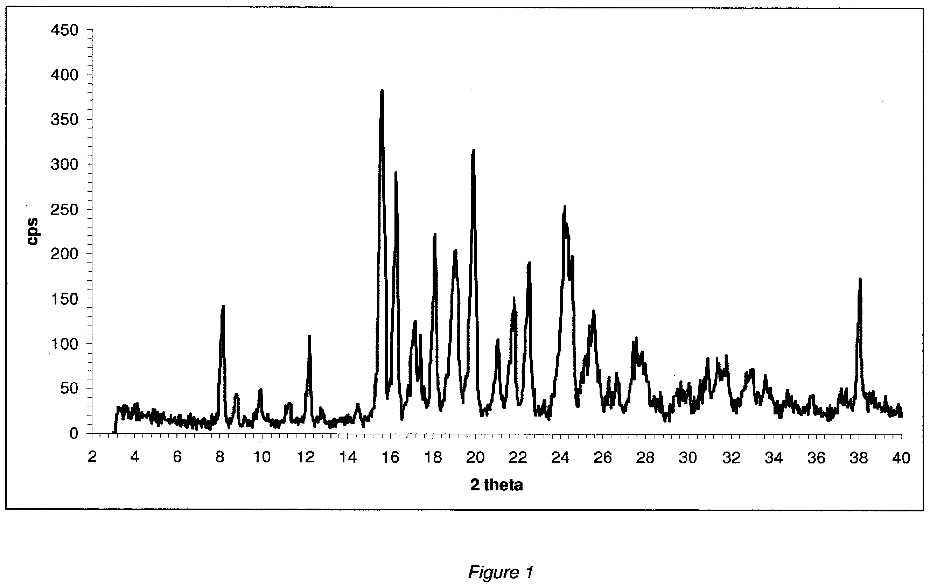

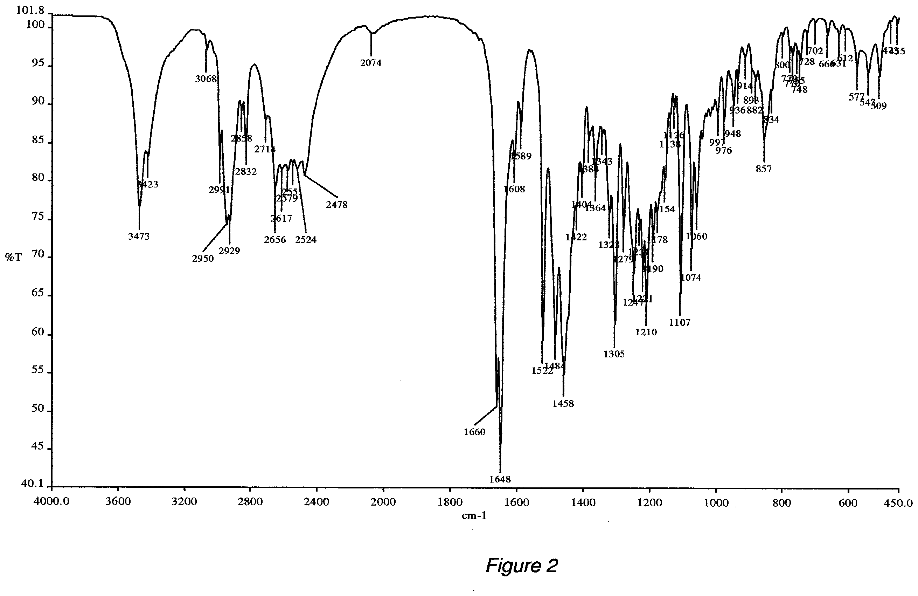

The powder diffraction spectrum and the FTIR spectrum characteristic of this form are depicted in Figures 1 and 2, respectively.

The present invention relates to methods for the preparation of the form ε of ivabradine hydrochloride.

A first method includes steps a) to c).

In step a) an anhydrous form of ivabradine hydrochloride is mixed with a C4-C7 ketone containing an amount of water between 0.1 and 1.5% by weight, preferably between 0.2 and 0.8%, for example 0.5%, and in a temperature range between - 10 and 50°C.

C4-C7 ketones suitable for the purpose are cyclic or acyclic ketones known and normally used in the field, such as, for example, 4-methylpentan-2-one (MIBK), cyclohexanone or preferably 3-pentanone (DEK) or 2-butanone (MEK). The amount of these solvents can vary in a very wide range; preferably, their volume may vary between 4 mL and 15 mL per gram of ivabradine hydrochloride used; even more preferably, the volume lies between 5 mL and 10 mL per gram of ivabradine hydrochloride; more concentrated solutions lead to a poorly stirrable mass, whereas more dilute solutions produce lower yields and greater economic and environmental impacts of the process.

Anhydrous forms of ivabradine hydrochloride that can be used in step a) include, for example, polymorphs α (alpha), δ (delta), δd (delta-d), I, ζ (Greek zed), X, or a mixture thereof.

In the subsequent step b) the mass thus produced is kept under stirring at a temperature between -10 and 50 °C, preferably between 20 and 30 °C, for a period of time between 1 and 50 hours, preferably between 2 and 25 hours. Where step b) is carried out at temperatures between 26 and 50 °C, the mass obtained in this step is cooled, or allowed to cool down, in a step b'), to a temperature between 20 and 25 °C, before proceeding to the next step c). Where, instead, step b) is carried out at temperatures between -10 and 25 °C, the subsequent step c) is carried out without changing the temperature of the system. In step c), the form ε of ivabradine hydrochloride is recovered as a solid. This step can be carried out using one of the methods known to those skilled in the art for the separation of a crystallized solid from the mother liquor, for example by filtration, optionally under pressure or *vacuum,* or by centrifugation.

The solid thus obtained is subsequently washed with at least one solvent, usually the same used in the previous crystallization step, and dried. Such drying can be carried out in a range of temperatures between 30 and 70 °C (preferably between 35 and 55 °C).

A second embodiment of the method of the present invention relates to a process for the preparation of the form ε of ivabradine hydrochloride including steps d) to g).

In step d), ivabradine free base is dissolved in a C4-C7 ketone containing an amount of water between 0.1 and 1.5% by weight, preferably between 0.2 and 0.8%, for example 0.5%, and in a temperature range between -10 and 50 °C.

C4-C7 ketones suitable for this purpose are the same as mentioned above in the description of step a). The amount of these solvents can vary in a very wide range, preferably between 3 mL and 15 mL per gram of ivabradine used; even more preferably said amount is between 5 mL and 10 mL per gram of ivabradine hydrochloride; more diluted solutions produce lower yields and greater economic and environmental impacts of the process.

The subsequent step e) consists in the preparation of ivabradine hydrochloride by treatment of the solution obtained in the previous step with hydrogen chloride.

In step f), the mass thus produced is kept under stirring at a temperature in the range between -10 and 50 °C, preferably between 20 and 30 °C, for a period of time between 5 and 50 hours, preferably between 10 and 20 hours.

Where step f) is carried out at temperatures between 26 and 50 °C, the mass obtained in this step is cooled, or allowed to cool down, in a step f), to a temperature between 20 and 25 °C, before proceeding to the next step g). Where, instead, step f) is carried out at temperatures between -10 and 25 °C, the subsequent step g) is carried out without changing the temperature of the system. The last step g) consists in the recovery of the solid and its subsequent drying between 30 and 70 °C with one of the techniques known to those skilled in the art, for example one of those described above to perform step c).

A variant of the process, subject of the third embodiment of the present invention includes an additional step, d'), carried out between steps d) and e), in which a seed of ivabradine hydrochloride form ε is added to the solution produced in step d), to promote the precipitation of this crystalline form. Preferably, the seed is added in a weight ratio ranging between 0.5% and 1.0% with respect to the amount of ivabradine used to form the solution of step d), and at the same temperature at which the solution of step d) was prepared.

The ε form of ivabradine hydrochloride obtained with the process of the present invention contains a percentage of water varying between 1.5 and 2% by weight.

The form ε of ivabradine hydrochloride obtained with the process of the present invention is particularly stable towards thermal stress and its preparation is particularly reproducible. Moreover, it can be prepared with processes cheap and effective, particularly suitable for large scale production. These processes can be used to obtain ivabradine hydrochloride with a chemical purity determined by HPLC greater than 99.5%, particularly suitable for the preparation of pharmaceutical dosage forms. This form of ivabradine hydrochloride can be used, in admixture with one or more pharmaceutically acceptable excipients, for the preparation of pharmaceutical formulations such as tablets, capsules, pills, powders, granules, pastilles, lozenges, elixirs, syrups, solutions, suspensions, emulsions, drops, lotions, sprays, tinctures, creams, pomades, gels, ointments, suppositories and transdermal devices for oral, enteral, parenteral or topical administration.

The present invention will be further illustrated through the following examples. XRPD analysis was carried out with a diffractometer APD 2000 Ital Structures operating at room temperature, using as a source of X-ray a CuKα tube (40 kV, 30 mA, λ = 1.5406 Å).

Acquisition was performed in step scan mode and in the Bragg-Brentano geometry, with steps of 0.04° per second over an interval 3-40° in theta/2theta. The samples were thoroughly ground in a mortar and placed in the recess of the aluminum sample holder. Before performing the measurement (remotely with WinAcq32 software), the instrument was calibrated with zinc oxide (purity > 99.5%).

The content of water present in the solvents used in the processes of the present invention or in form ε of ivabradine hydrochloride resulting therefrom was determined by Karl Fischer analysis by means of a Mettler-Toledo DL38 automatic titrator, using Combititrant-5 as titrating medium (1 ml correspond to 5 mg of water) and Hydranal Ketosolver as a titration solvent.

The DSC analyses were performed with a Mettler-Toledo Star^{e} differential scanning calorimeter, in perforated aluminum crucibles, heating the sample from 30 to 300 °C in nitrogen atmosphere.

The IR analysis was performed with a Perkin Elmer SPECTRUM ONE FT-IR spectrometer dispersing the samples in a KBr pellet. Measurement was performed by carrying out 32 scans with a resolution of 4.0 cm⁻¹ in a range between 4000 and 400 cm⁻¹.

### Example 1

### Preparation of form ε of ivabradine hydrochloride starting from form δ

The form δ of ivabradine hydrochloride (3.0 g, 5.9 mmol) is suspended, under magnetic stirring and at 25 °C, in 3-pentanone (15 mL) containing 0.5% by weight of water. The mass is kept under stirring at the same temperature for 6 hours, then the obtained solid is filtered, washed with 3-pentanone and dried at 45 °C and under reduced pressure. 2.4 g of the form ε of ivabradine hydrochloride are thus obtained, characterized by an XRPD spectrum as depicted in Figure 1.

### Example 2

### Preparation of form ε of ivabradine hydrochloride starting from form α

The form α of ivabradine hydrochloride (1.0 g, 2.0 mmol) is suspended, under magnetic stirring and at 25 °C, in 3-pentanone (5 mL) containing 0.5% by weight of water. The mass is kept under stirring at the same temperature for 48 hours, then the obtained solid is filtered, washed with 3-pentanone and dried at 45 °C and under reduced pressure. 810 mg of the form ε of ivabradine hydrochloride are thus obtained, characterized by an XRPD spectrum corresponding to that obtained in example 1.

### Example 3

### Preparation of form ε of ivabradine hydrochloride starting from form δd

The form δd of ivabradine hydrochloride (3.0 g, 5.9 mmol) is suspended, under magnetic stirring and at 25 °C, in 3-pentanone (15 mL) containing 0.5% by weight of water. The mass is kept under stirring at the same temperature for 4 hours, then the obtained solid is filtered, washed with 3-pentanone and dried at 45 °C and under reduced pressure. 2.3 g of the form ε of ivabradine hydrochloride are thus obtained, characterized by an XRPD spectrum corresponding to that obtained in example 1.

### Example 4

### Preparation of form ε of ivabradine hydrochloride starting from form I

The form I of ivabradine hydrochloride (3.0 g, 5.9 mmol) (prepared according to the procedure described in example 3 of international application WO 2008/065681) is suspended, under magnetic stirring and at 25 °C, in 3-pentanone (15 mL) containing 0.5% by weight of water. The mass is kept under stirring at the same temperature for 3 hours, then the obtained solid is filtered, washed with 3-pentanone and dried at 45 °C and under reduced pressure. 2.6 g of the form ε of ivabradine hydrochloride are thus obtained, characterized by an XRPD spectrum corresponding to that obtained in example 1.

### Example 5

### Preparation of form ε of ivabradine hydrochloride starting from form ζ (Greek zed)

The form ζ of ivabradine hydrochloride (3.0 g, 5.9 mmol) (prepared according to the procedure described in example 3 of international application WO 2012/025940) is suspended, under magnetic stirring and at 25 °C, in 3-pentanone (15 mL) containing 0.5% by weight of water. The mass is kept under stirring at the same temperature for 2 hours, then the obtained solid is filtered, washed with 3-pentanone and dried at 45 °C and under reduced pressure. 2.4 g of the form ε of ivabradine hydrochloride are thus obtained, characterized by an XRPD spectrum corresponding to that obtained in example 1.

### Example 6

### Preparation of form ε of ivabradine hydrochloride starting from basic ivabradine

Ivabradine free base (8.0 g, 17.1 mmol) is solubilized, under magnetic stirring and at 25 °C, in 2-butanone (80 mL) containing 0.5% by weight of water, then hydrogen chloride is bubbled until a pH of about 1 has been reached, as measured with a litmus test moistened with water. The reaction mass is kept under stirring for 33 hours at 25 °C, then the obtained solid is filtered, washed with 2-butanone and dried at 45 °C and under reduced pressure. 8.1 g of the form ε of ivabradine hydrochloride are thus obtained, characterized by an XRPD spectrum corresponding to that obtained in example 1.

### Example 7

### Preparation of form ε of ivabradine hydrochloride starting from form δ

The form δ of ivabradine hydrochloride (2.0 g, 4.0 mmol) is suspended, under magnetic stirring and at 0 °C, in 3-pentanone (10 mL) containing 0.5% by weight of water. The mass is kept under stirring at the same temperature for 26 hours, then filtered. The solid thus obtained is washed with 3-pentanone and dried at 45 °C and under reduced pressure. 1.5 g of the form ε of ivabradine hydrochloride are thus obtained, characterized by an XRPD spectrum corresponding to that obtained in example 1.

### Example 8

### Preparation of form ε of ivabradine hydrochloride starting from form δ

The form δ of ivabradine hydrochloride (2.0 g, 4.0 mmol) is suspended, under magnetic stirring and at 25 °C, in 3-pentanone (20 mL) containing 0.5% by weight of water. The mass is kept under stirring at the same temperature for 3 hours, then the obtained solid is filtered, washed with 3-pentanone and dried at 45 °C and under reduced pressure. 1.6 g of the form ε of ivabradine hydrochloride are thus obtained, characterized by an XRPD spectrum corresponding to that obtained in example 1.

### Example 9

### Preparation of form ε of ivabradine hydrochloride starting from form δ

The form δ of ivabradine hydrochloride (2.0 g, 4.0 mmol) is suspended, under magnetic stirring and at 25 °C, in 3-pentanone (8 mL) containing 0.5% by weight of water. The mass is kept under stirring at the same temperature for 4 hours, then the obtained solid is filtered, washed with 3-pentanone and dried at 45 °C and under reduced pressure. 1.6 g of the form ε of ivabradine hydrochloride are thus obtained, characterized by an XRPD spectrum corresponding to that obtained in example 1.

### Example 10

### Preparation of form ε of ivabradine hydrochloride starting from form X

The form X of ivabradine hydrochloride (3.0 g, 5.9 mmol) (prepared according to the procedure described in example 3 of international application WO 2011/098582) is suspended, under magnetic stirring and at 25 °C, in 3-pentanone (15 mL) containing 0.5% by weight of water. The mass is kept under stirring at the same temperature for 2 hours, then the obtained solid is filtered, washed with 3-pentanone and dried at 45 °C and under reduced pressure. 2.6 g of the form ε of ivabradine hydrochloride are thus obtained, characterized by an XRPD spectrum corresponding to that obtained in example 1.

### Example 11

### Preparation of form ε of ivabradine hydrochloride starting from form δ

The form δ of ivabradine hydrochloride (2.0 g, 4.0 mmol) is suspended, under magnetic stirring and at 25 °C, in 2-butanone (20 mL) containing 1% by weight of water. The mass is kept under stirring at the same temperature for 6 hours, then the obtained solid is filtered, washed with 2-butanone and dried at 45 °C and under reduced pressure. 1.7 g of the form ε of ivabradine hydrochloride are thus obtained, characterized by an XRPD spectrum corresponding to that obtained in example 1.

### Example 12

### Preparation of form ε of ivabradine hydrochloride starting from form δ

The form δ of ivabradine hydrochloride (2.0 g, 4.0 mmol) is suspended, under magnetic stirring and at 25 °C, in 2-butanone (20 mL) containing 1.5% by weight of water. The mass is kept under stirring at the same temperature for 6 hours, then the obtained solid is filtered, washed with 2-butanone and dried at 45 °C and under reduced pressure. 1.6 g of the form ε of ivabradine hydrochloride are thus obtained, characterized by an XRPD spectrum corresponding to that obtained in example 1.

### Example 13

### Preparation of form ε of ivabradine hydrochloride starting from form δ

The form δ of ivabradine hydrochloride (2.0 g, 4.0 mmol) is suspended, under magnetic stirring and at 40 °C, in 3-pentanone (10 mL) containing 0.5% by weight of water. The mass is kept under stirring at the same temperature for 3 hours, then cooled down to 25 °C and filtered. The solid thus obtained is washed with 3-pentanone and dried at 45 °C and under reduced pressure. 1.6 g of the form ε of ivabradine hydrochloride are thus obtained, characterized by an XRPD spectrum corresponding to that obtained in example 1.

### Example 14

### Preparation of form ε of ivabradine hydrochloride starting from form δ

The form δ of ivabradine hydrochloride (2.0 g, 4.0 mmol) is suspended, under magnetic stirring and at 25 °C, in 2-butanone (20 mL) containing 0.5% by weight of water. The mass is kept under stirring at the same temperature for 2 hours, then the obtained solid is filtered, washed with 2-butanone and dried at 45 °C and under reduced pressure. 1.7 g of the form ε of ivabradine hydrochloride are thus obtained, characterized by an XRPD spectrum corresponding to that obtained in example 1.

### Example 15

### Preparation of form ε of ivabradine hydrochloride starting from form δ

The form δ of ivabradine hydrochloride (2.0 g, 4.0 mmol) is suspended, under magnetic stirring and at 25 °C, in 4-methylpentan-2-one (20 mL) containing 0.5% by weight of water. The mass is kept under stirring at the same temperature for 2 hours, then the obtained solid is filtered, washed with 4-methylpentan-2-one and dried at 45 °C and under reduced pressure. 1.6 g of the form ε of ivabradine hydrochloride are thus obtained, characterized by an XRPD spectrum corresponding to that obtained in example 1.

### Example 16

### Preparation of form ε of ivabradine hydrochloride starting from form δd

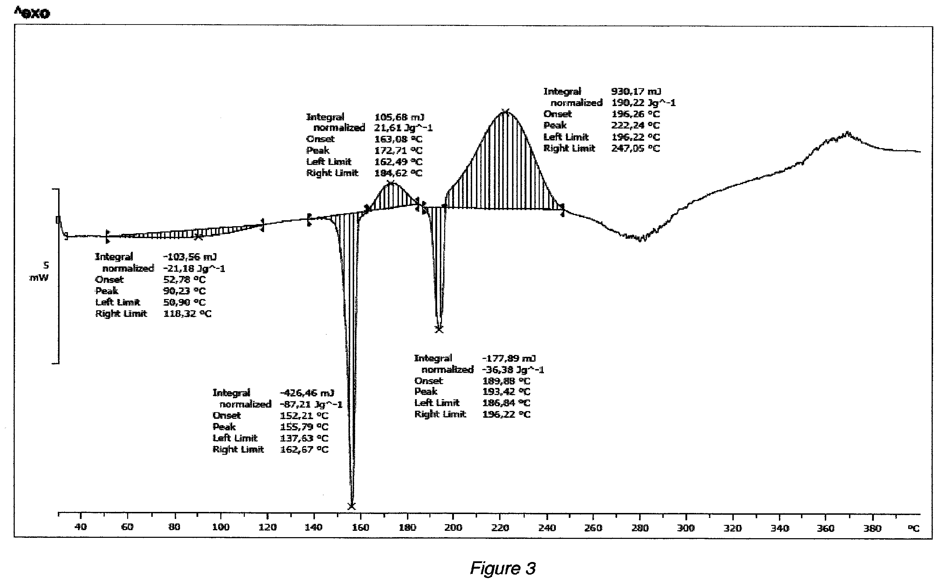

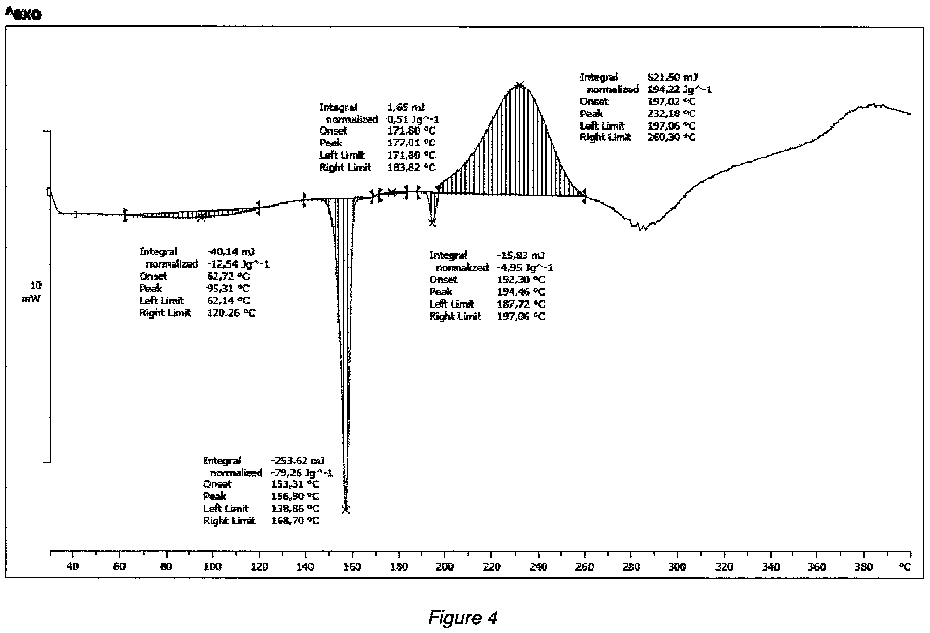

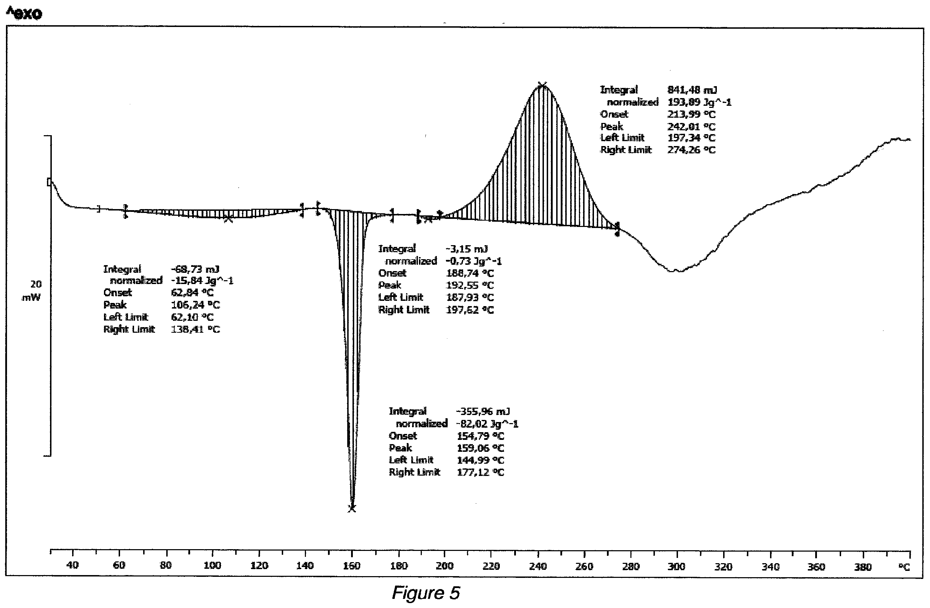

The form δd of ivabradine hydrochloride (18.4 g, 36.4 mmol) is suspended, under magnetic stirring and at 25 °C, in 3-pentanone (92 mL) containing 0.5% by weight of water. The mass is kept under stirring at the same temperature for 4 hours, then the obtained solid is filtered, washed with 3-pentanone and dried at 35 °C and under reduced pressure. 16.7 g of the form ε of ivabradine hydrochloride are thus obtained, characterized by an XRPD spectrum corresponding to that obtained in example 1. The obtained solid is analyzed by DSC with heating ramps of 5, 10 and 20 °C per minute; the results of the three tests are depicted in Figures 3, 4, and 5 respectively.

### Example 17

### Preparation of form ε of ivabradine hydrochloride starting from form δ

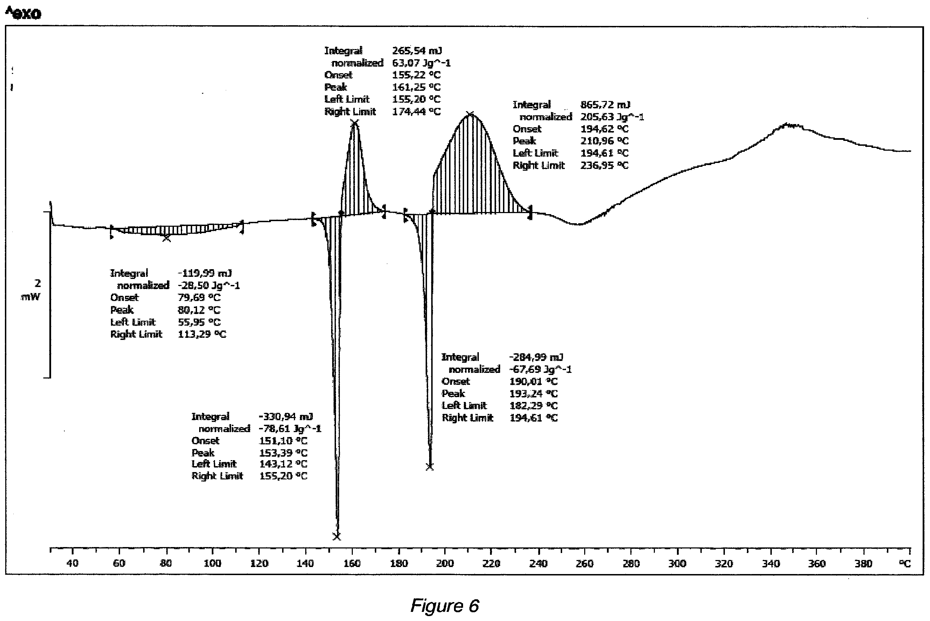

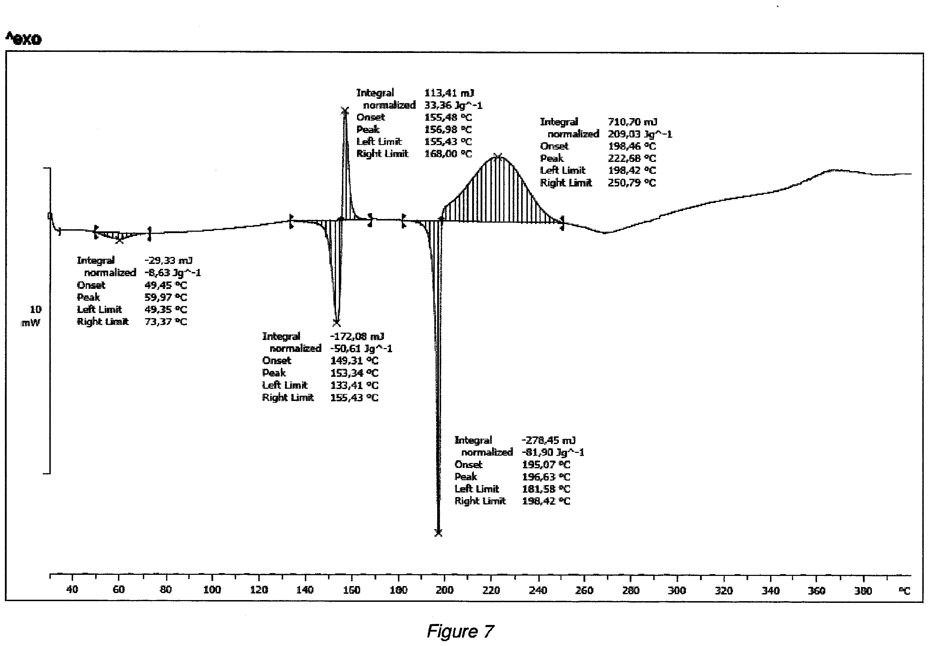

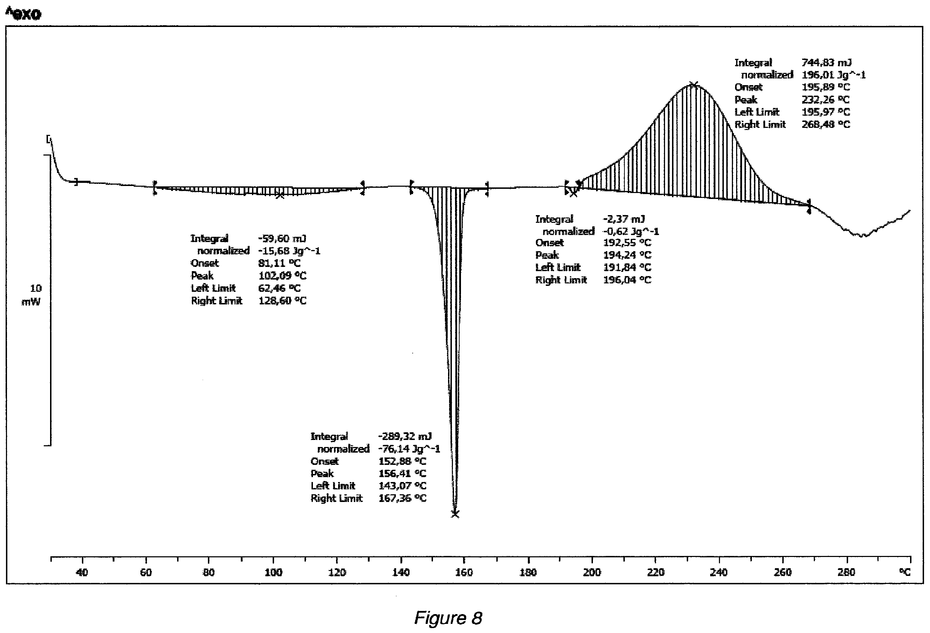

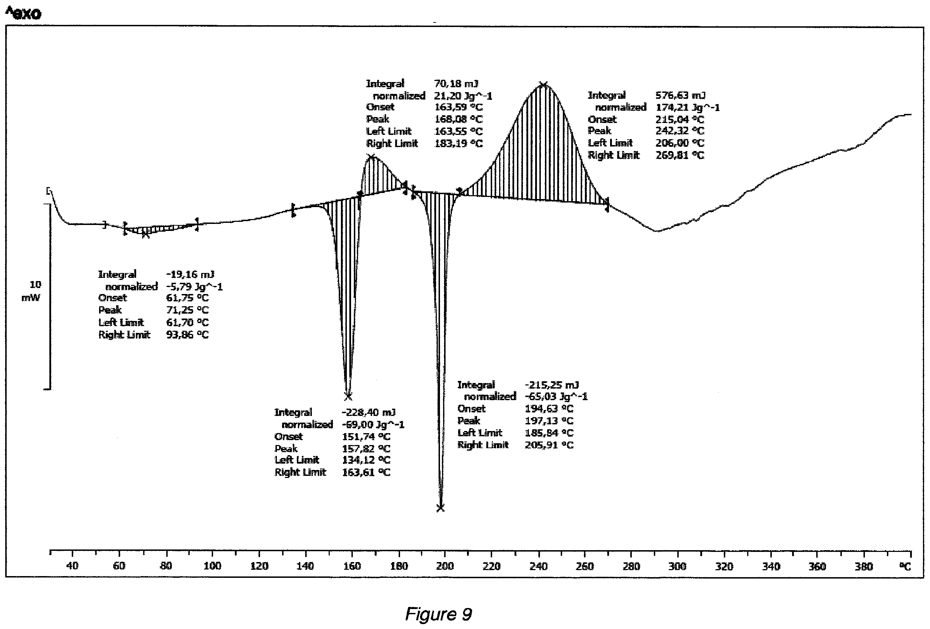

The form δ of ivabradine hydrochloride (41.4 g, 82.0 mmol) is added, under magnetic stirring and at 25 °C, to 2-butanone (414 mL) containing 0.5% by weight of water, in about 5 minutes. The mass is kept under stirring at the same temperature for 2 hours, then the obtained solid is filtered, washed with 2-butanone and dried at 55 °C and under reduced pressure. 37.6 g of the form ε of ivabradine hydrochloride are thus obtained, characterized by an XRPD spectrum corresponding to that obtained in example 1. The solid obtained is analyzed by DSC with heating ramps of 2, 5, 10, and 20 °C per minute; the results of the four tests are depicted in Figures 6, 7, 8 and 9 respectively.

### Example 18

### Analysis of the thermal stability of the polymorph ε of ivabradine hydrochloride prepared according to the processes of the invention

The form ε of ivabradine hydrochloride (2.0 g, 4.0 mmol), prepared, for example, as described in Example 1, is maintained at 100 °C under *vacuum* for 12 hours. The solid is then brought to room temperature and subjected to XRPD analysis, giving rise to a XRPD spectrum corresponding to that obtained in example 1.

### Example 19 (Comparative)

### Repetition of example 10 of WO 2013/064307

The form α of ivabradine hydrochloride (10 mg) is suspended, under magnetic stirring and at 20-25 °C, in 4-methylpentan-2-one (1.5 mL). The mass is kept under stirring at the same temperature for 3 days, then the obtained solid is filtered and dried under *vacuum.* The solid obtained is analyzed by XRPD yielding to a spectrum consistent to crystalline form α.

### Example 20 (Comparative)

### Repetition of example 15 of WO 2013/064307

The form δ of ivabradine hydrochloride (20.6 mg) is suspended, under magnetic stirring and at 20-25 °C, in 4-methylpentan-2-one (1 mL). The mass is kept under stirring at the same temperature for 4 days, then the obtained solid is filtered and dried under *vacuum.* The solid obtained is analyzed by XRPD yielding to a spectrum consistent to crystalline form α.

### Comments to the Examples

Examples 19 and 20 have been carried out in order to compare the processes of the present invention with those described in international application WO 2013/064307 (hereinafter WO '307), published after the priority dates of the present application. WO '307 describes three different procedures to prepare an ivabradine hydrochloride crystalline form, therein referred to as form IV, using a mixture of:
- crystalline form ivabradine hydrochloride as obtained following the procedure of EP 0534859 and with a water content of 1.6% by weight in a series of solvents comprising toluene, toluene/acetone (10:1), toluene/ethyl acetate (10:1), ethyl acetate/ethanol (9:1), isopropyl acetate;
- crystalline ivabradine hydrochloride forms α, α+δd, δ in a series of solvent comprising, among others, 4-methylpentan-2-one and 2-butanone;
- a mixture of crystalline forms α and IV in a series of solvents comprising 4-methylpentan-2-one and 2-butanone.

The last two processes could be considered similar to the processes described in the present application for the preparation of crystalline form ε.

Comparative Examples 19 and 20 have been carried out repeating some of the procedures disclosed in WO '307 involving the use of 4-methylpentan-2-one or 2-butanone (C4-C7 ketones) to prepare polymorphic form IV, which, in the opinion of the present inventors, can be considered a fair representation of all examples involving the use of ketones.

As a result of these comparative tests, the present inventors have found that the procedures described in WO '307 do not actually lead to crystalline forms IV (claimed in WO '307) or ε of ivabradine hydrochloride (as described in the present application), but rather to crystalline form α. As a further remark, it is noted that the processes of WO '307 entailing the use of ketones do not disclose the processes or the form described in the present application either explicitly (for example due to the lack of the water range in the solvents to be used) or implicitly (yielding to form α of ivabradine hydrochloride).

## Claims

1. A method for the preparation of polymorph ε (epsilon) of ivabradine hydrochloride, compound having the structural formula below: 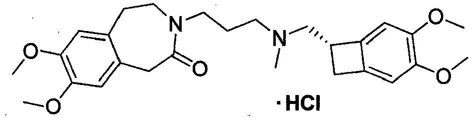 containing water in an amount between 1.5 and 2% by weight and **characterized by** a XRPD profile that, when collected with Kα radiation of copper, comprises peaks at 8.1°, 12.2°, 15.6°, 18.1°, 19.9°, 24.2° and 31.7° 2θ, said method including the following steps:
a) mixing anhydrous ivabradine hydrochloride and a C4-C7 ketone containing an amount of water between 0.1 and 1.5% by weight;
b) keeping under stirring the bulk for a period of time between 1 and 50 hours at a temperature between -10 and 50 °C;
c) recovering the resulting solid;
c') washing the recovered solid with at least one solvent and drying it between 30 and 70 °C;
wherein the anhydrous ivabradine hydrochloride is in the form of one of the polymorphs α (alpha), δ (delta), δd (delta-d), I, ζ (Greek zed), X, or a mixture thereof.

2. Method according to claim 1, wherein the amount of water contained in said ketone is between 0.2 and 0.8% by weight.

3. Method according to any one of claims 1 or 2, wherein in step a) between 4 mL and 15 mL of said ketone are used per gram of ivabradine hydrochloride.

4. Method according to any one of claims 1 to 3, wherein step b) is carried out at a temperature between 20 and 30 °C.

5. Method according to any one of claims 1 to 4, wherein, if step b) is carried out at a temperature above 26 °C, a further step b') is carried out between steps b) and c), in which the bulk obtained in step b) is cooled or allowed to cool down to a temperature between 20 and 25 °C.

6. A method for the preparation of polymorph ε (epsilon) of ivabradine hydrochloride, compound having the structural formula below: 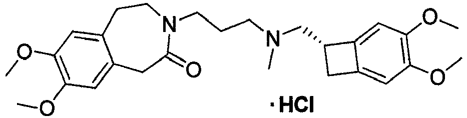 containing water in an amount between 1.5 and 2% by weight and **characterized by** a XRPD profile that, when collected with Kα radiation of copper, comprises peaks at 8.1°, 12.2°, 15.6°, 18.1°, 19.9°, 24.2° and 31.7° 2θ, said method including the following steps:
d) solubilizing free base ivabradine in a C4-C7 ketone containing an amount of water between 0.1 and 1.5% by weight;
e) treating with hydrogen chloride;
f) keeping under stirring the bulk obtained in step e) for a period of time between 5 and 50 hours at a temperature between -10 and 50 °C;
g) recovering the resulting solid;
h) washing the recovered solid with at least one solvent and drying it between 30 and 70 °C.

7. Method according to claim 6, wherein the amount of water contained in said ketone is between 0.2 and 0.8% by weight.

8. Method according to any one of claims 6 or 7, wherein in step d) between 3 mL and 15 mL of said ketone are used per gram of ivabradine.

9. Method according to any one of claims 6 to 8, wherein step f) is carried out at a temperature between 20 and 30 °C.

10. Method according to any one of claims 6 to 9, wherein, if step f) is carried out at a temperature above 26 °C, a further step f) is carried out between steps f) and g), in which the bulk obtained in step f) is cooled or allowed to cool down to a temperature between 20 and 25 °C.

11. Method according to claims 1 or 6 wherein the ketone used in steps a) or d) is selected among 4-methylpentan-2-one, cyclohexanone, 3-pentanone and 2-butanone.

## Patentansprüche

1. Verfahren zur Herstellung eines Polymorphs ε (epsilon) der Verbindung Ivabradin-Hydrochlorid, welche die untenstehende Strukturformel aufweist: 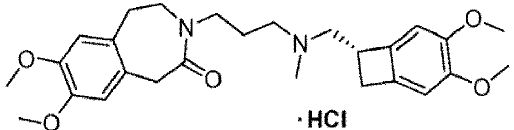 , welches Wasser in einer Menge zwischen 1,5 und 2 Gewichtsprozent enthält und **gekennzeichnet durch** ein XRPD-Profil, das bei Aufnahme mit Kupfer-Kα-Strahlung Peaks bei 8,1°, 12,2°, 15,6°, 18,1°, 19,9°, 24,2° und 31,7° 2θ umfasst, umfassend die folgenden Schritte:
a) das Vermischen von wasserfreiem Ivabradin-Hydrochlorid und einem C4-C7-Keton, das eine Menge an Wasser zwischen 0,1 und 1,5 Gewichtsprozent enthält;
b) das beständige Rühren der Masse für einen Zeitraum zwischen 1 und 50 Stunden bei einer Temperatur zwischen -10 und 50 °C;
c) das Gewinnen des resultierenden Feststoffes
c') Waschen des gewonnenen Feststoffes mit mindestens einem Lösemittel und dessen Trocknung zwischen 30 und 70 °C;
wobei das wasserfreie Ivabradin-Hydrochlorid in Gestalt eines der Polymorphen α (alpha), δ (delta), δd (delta-d), I, ζ (zeta), X oder einer Mischung derselben vorliegt.

2. Verfahren nach Anspruch 1, wobei die in dem Keton enthaltene Menge an Wasser zwischen 0,2 und 0,8 Gewichtsprozent liegt.

3. Verfahren nach einem der Ansprüche 1 oder 2, wobei in Schritt a) zwischen 4 mL und 15 mL des Ketons pro Gramm Ivabradin-Hydrochlorid verwendet werden.

4. Verfahren nach einem der Ansprüche 1 bis 3, wobei Schritt b) bei einer Temperatur zwischen 20 und 30 °C durchgeführt wird.

5. Verfahren nach einem der Ansprüche 1 bis 4, wobei, wenn Schritt b) bei einer Temperatur über 26 °C durchgeführt wird, zwischen den Schritten b) und c) ein zusätzlicher Schritt b') durchgeführt wird, in welchem man die in Schritt b) erhaltene Masse auf eine Temperatur zwischen 20 und 25 °C abkühlt oder abkühlen lässt.

6. Verfahren zur Herstellung des Polymorphs ε (epsilon) der Verbindung Ivabradin-Hydrochlorid, welche die untenstehende Strukturformel aufweist: 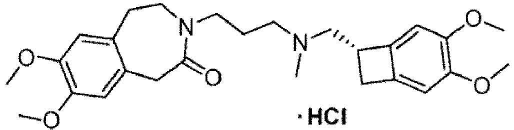 , welches Wasser in einer Menge zwischen 1,5 und 2 Gewichtsprozent enthält und **gekennzeichnet durch** ein XRPD-Profil, das bei Aufnahme mit Kupfer-Kα-Strahlung Peaks bei 8,1°, 12,2°, 15,6°, 18,1°, 19,9°, 24,2° und 31,7° 2θ umfasst, umfassend die folgenden Schritte:
d) das Solubilisieren der freien Base Ivabradin in einem C4-C7-Keton, das eine Menge an Wasser zwischen 0,1 und 1,5 Gewichtsprozent enthält;
e) das Behandeln mit Chlorwasserstoff;
f) das beständige Rühren der in Schritt e) erhaltenen Masse für einen Zeitraum zwischen 5 und 50 Stunden bei einer Temperatur zwischen -10 und 50 °C;
g) das Gewinnen des resultierenden Feststoffes und dessen Trocknung zwischen 30 und 70 °C.
h) Waschen des gewonnenen Feststoffes mit mindestens einem Lösemittel.

7. Verfahren nach Anspruch 6, wobei die in dem Keton enthaltene Menge an Wasser zwischen 0,2 und 0,8 Gewichtsprozent liegt.

8. Verfahren nach einem der Ansprüche 6 oder 7, wobei in Schritt d) zwischen 3 mL und 15 mL des Ketons pro Gramm Ivabradin verwendet werden.

9. Verfahren nach einem der Ansprüche 6 bis 8, wobei Schritt f) bei einer Temperatur zwischen 20 und 30 °C durchgeführt wird.

10. Verfahren nach einem der Ansprüche 6 bis 9, wobei, wenn Schritt f) bei einer Temperatur über 26 °C durchgeführt wird, zwischen den Schritten f) und g) ein zusätzlicher Schritt f') durchgeführt wird, in welchem man die in Schritt f) erhaltene Masse auf eine Temperatur zwischen 20 und 25 °C abkühlt oder abkühlen lässt.

11. Verfahren nach Anspruch 1 oder 6, wobei das in den Schritten a) oder d) verwendete Keton ausgewählt ist aus 4-Methylpentan-2-on, Cyclohexanon, 3-Pentanon und 2-Butanon.

## Revendications

1. Procédé de préparation d'un polymorphe ε (epsilon) de chlorhydrate d'ivabradine, composé ayant la formule structurale ci-dessous : 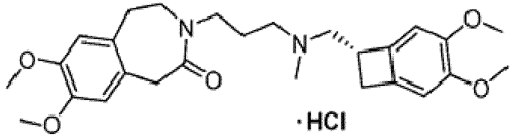 contenant de l'eau en une quantité comprise entre 1,5 et 2% en poids et **caractérisé par** un profil XRPD qui, lorsqu'il est collecté avec le rayonnement Ka de cuivre, comprend des pics à 8.1°, 12.2°, 15.6°, 18.1°, 19.9°, 24.2° et 31.7° 2θ, ledit procédé incluant les étapes suivantes :
a) mélange de chlorhydrate d'ivabradine anhydre et d'une cétone en C4-C7 contenant une quantité d'eau entre 0,1 et 1,5 % en poids ;
b) maintien sous agitation de la masse pendant une période de temps entre 1 et 50 heures à une température entre -10 et 50 °C ;
c) récupération du solide ;
c') lavage du solide récupéré avec au moins un solvant et séchage de celui-ci entre 30 et 70 °C ;
dans lequel le chlorhydrate d'ivabradine anhydre se trouve sous la forme de l'un des polymorphes α (alpha), δ (delta), δd (delta-d), I, ζ (z grec), X, ou d'un mélange de ceux-ci.

2. Procédé selon la revendication 1, dans lequel la quantité d'eau contenue dans ladite cétone est entre 0,2 et 0,8 % en poids.

3. Procédé selon l'une quelconque des revendications 1 ou 2, dans lequel à l'étape a) entre 4 mL et 15 mL de ladite cétone sont utilisés par gramme de chlorhydrate d'ivabradine.

4. Procédé selon l'une quelconque des revendications 1 à 3, dans lequel l'étape b) est réalisée à une température entre 20 et 30 °C.

5. Procédé selon l'une quelconque des revendications 1 à 4, dans lequel si l'étape b) est réalisée à une température supérieure à 26 °C, une étape supplémentaire b') est réalisée entre les étapes b) et c), au cours de laquelle la masse obtenue à l'étape b) est refroidie ou a le temps de refroidir à une température entre 20 et 25 °C.

6. Procédé de préparation de polymorphe ε (epsilon) de chlorhydrate d'ivabradine, composé ayant la formule structurale ci-dessous : 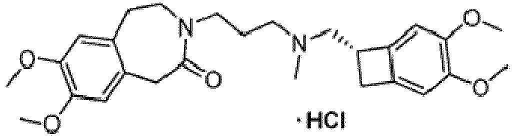 contenant de l'eau en une quantité comprise entre 1,5 et 2% en poids et **caractérisé par** un profil XRPD qui, lorsqu'il est collecté avec le rayonnement Ka de cuivre, comprend des pics à 8.1°, 12.2°, 15.6°, 18.1 , 19.9°, 24.2° et 31.7° 2θ, ledit procédé incluant les étapes suivantes :
d) solubilisation de l'ivabradine base libre dans une cétone en C4-C7 contenant une quantité d'eau entre 0,1 et 1,5 % en poids ;
e) traitement avec du chlorure d'hydrogène ;
f) maintien sous agitation de la masse obtenue à l'étape e) pour une période de temps entre 5 et 50 heures à une température entre -10 et 50 °C ;
g) récupération du solide
h) lavage du solide récupéré avec au moins un solvant et séchage de celui-ci entre 30 et 70 °C.

7. Procédé selon la revendication 6, dans lequel la quantité d'eau contenue dans ladite cétone est entre 0,2 et 0,8 % en poids.

8. Procédé selon l'une quelconque des revendications 6 ou 7, dans lequel à l'étape d) entre 3 mL et 15 mL de ladite cétone sont utilisés par gramme d'ivabradine.

9. Procédé selon l'une quelconque des revendications 6 à 8, dans lequel l'étape f) est réalisée à une température entre 20 et 30 °C.

10. Procédé selon l'une quelconque des revendications 6 à 9, dans lequel, si l'étape f) est réalisée à une température supérieure à 26 °C, une étape supplémentaire f') est réalisée entre les étapes f) et g), au cours de laquelle la masse obtenue à l'étape f) est refroidie ou a le temps de refroidir à une température entre 20 et 25 °C.

11. Procédé selon les revendications 1 ou 6 dans lequel la cétone utilisée aux étapes a) ou d) est sélectionnée parmi la 4-méthylpentan-2-one, la cyclohexanone, la 3-pentanone et la 2-butanone.
